Europäisches Patentamt

European Patent Office

Office européen des brevets

(11) Veröffentlichungsnummer: **0 310 740**
**A2**

(12) # EUROPÄISCHE PATENTANMELDUNG

(21) Anmeldenummer: **88103840.0**

(22) Anmeldetag: **11.03.88**

(51) Int. Cl.⁴: **G01N 21/85 , G01N 35/08**

(30) Priorität: **03.10.87 DE 3733573**

(43) Veröffentlichungstag der Anmeldung:
**12.04.89 Patentblatt 89/15**

(84) Benannte Vertragsstaaten:
**DE FR GB NL SE**

(71) Anmelder: **LEYBOLD AKTIENGESELLSCHAFT**
**Wilhelm-Rohn-Strasse 25**
**D-6450 Hanau 1(DE)**

(72) Erfinder: **Mayer, Jürgen**
**Hasenpfad 1**
**D-6450 Hanau(DE)**

(54) **Vorrichtung zum Messen des Fremdstoffanteils in strömenden Flüssigkeiten.**

(57) Bei einer Vorrichtung zur Bestimmung der Konzentration von Fremdstoffen in einem Wasserstrom wird jeweils vor und nach dem Leitungsblock (4) für den Prozeß ein Flüssigkeitsstrom über Leitungen (8, 9) abgezweigt und jeweils in eine Küvette (16, 17) gefördert, wobei den Küvetten (16, 17) jeweils eine Strahlenquelle (18) zugeordnet ist, deren Strahlenbündel die Küvetten (16, 17) durchdringen und von einem Strahlenteiler jeweils sowohl einem ersten Detektor als auch einem zweiten Detektor zugeleitet werden. Jedem der Detektoren (19) ist jweils ein Strahlenfilter vorgeschaltet, das die Strahlen in zwei Strahlenbündel unterschiedlicher Wellenlängen aufteilt. Zwischen Strahlenquelle (18) und den beiden Küvetten (16, 17) ist ein rotierendes Chopper-Rad angeordnet, das die Strahlen der Strahlenquelle (24) abwechselnd in die eine und die andere Küvette (16, 17) lenkt, wobei die elektrischen Signale der Detektoren (19) von einer elektrischen Schaltung miteinander verglichen und zu einem Erkennungssignal verarbeitet werden.

## FIG. 1

## Vorrichtung zum Messen des Fremdstoffanteils in strömenden Flüssigkeiten

Die Erfindung betrifft eine Vorrichtung zum Messen des Fremdstoffanteils in strömenden Flüssigkeiten, insbesondere zur Bestimmung der Konzentration von aromatischen Kohlenwasserstoffen in einem Wasserstrom.

Es sind Vorrichtungen zum Messen der Fremdstoffkonzentration in einem Gasstrom bekannt, die der Prozeßkontrolle in Chemieanlagen, zur Rauchgasmessung und zur Emissionsmessung an Feuerungsanlagen und an Kraftfahrzeugen dienen. Diese bekannte Vorrichtung arbeitet nach dem nichtdispersiven Ultraviolett-Absorptions-Meßverfahren. Als Meßeffekt dient die spezifische Strahlungsabsorption der Meßkomponente im UV-Bereich.

Die UV-Strahlung wird in einer Hohlkathodenlampe erzeugt. Ein Blendenrad teilt die Strahlung in zwei zeitlich getrennte und ein Strahlenteiler in zwei räumlich getrennte Strahlungen. Auf einen Empfänger trifft dabei der Meßstrahl, der durch die Meßküvette geleitet wird. Der völlig unbeeinflußte Vergleichsstrahl trifft auf den Korrekturempfänger.

Die elektronische Verarbeitung dieser 4 Signale eliminiert die Einflußeffekte von nichtselektiven Absorptionen, wie zum Beispiel Küvettenverschmutzung und Alterungseffekte von Strahler und Empfänger.

Der vorliegenden Erfindung liegt die Aufgabe zugrunde, eine Vorrichtung zu schaffen, die im Durchflußbetrieb eine Vergleichsmessung zwischen beströmter Referenz und Probe durchführt und die geeignet ist, bei Überschreiten einer vorbestimmten Konzentration des Fremdstoffs einen Grenzwert-Kontakt zu schalten.

Erfindungsgemäß wird diese Aufgabe dadurch gelöst, daß jeweils vor und nach dem Leitungsblock für den Prozeß ein Flüssigkeitsstrom über Leitungen abgezweigt und jeweils in eine Küvette gefördert wird, wobei den beiden Küvetten jeweils eine Strahlenquelle zugeordnet ist, deren Strahlenbündel die Küvetten durchdringen und von einem Strahlenteiler jeweils sowohl einem ersten Detektor als auch einem zweiten Detektor zugeleitet werden, wobei jedem der Detektoren jeweils ein Strahlenfilter vorgeschaltet ist, das die Strahlen in zwei Strahlenbündel unterschiedlicher Wellenlänge aufteilt, und wobei zwischen Strahlenquelle und den beiden Küvetten ein rotierendes Chopper-Rad angeordnet ist, das die Strahlen der Strahlenquelle abwechselnd in die eine und die andere Küvette lenkt, wobei die elektrischen Signale der Detektoren von einer elektrischen Schaltung miteinander verglichen und zu einem Erkennungssignal verarbeitet werden.

Weitere Merkmale, Einzelheiten und Ausführungsformen der Erfindung ergeben sich aus den anhängenden Patentansprüchen.

Der durch die Erfindung erreichte Vorteil ist im wesentlichen dain zu sehen, daß bei einer großtechnischen Anlage während eines laufenden Prozesses eine kontinuierliche Überwachung des Ausgangswassers oder Abwassers während eines langen Zeitraumes durchgeführt werden kann, wobei der Anteil der Verschmutzung des Eingangswassers erkannt und vom Anteil der durch den Prozeß erzeugten Verschmutzung unterschieden wird.

Die Erfindung läßt die verschiedensten Ausführungsmöglichkeiten zu; eine davon ist in den anhängenden Zeichnungen schematisch näher dargestellt:

Figur 1 das Schaltbild für eine Vorrichtung zur laufenden Ermittlung des Benzol-Anteils in durchfließendem Wasser

Figur 2 den Aufbau der Meßeinrichtung der Vorrichtung gemäß Figur 1

Figur 3 die Küvette der Meßeinrichtung gemäß Figur 1

Figur 4 die Darstellung des an die Detektoren der Meßeinrichtung angeschlossenen elektrischen Schaltkreises

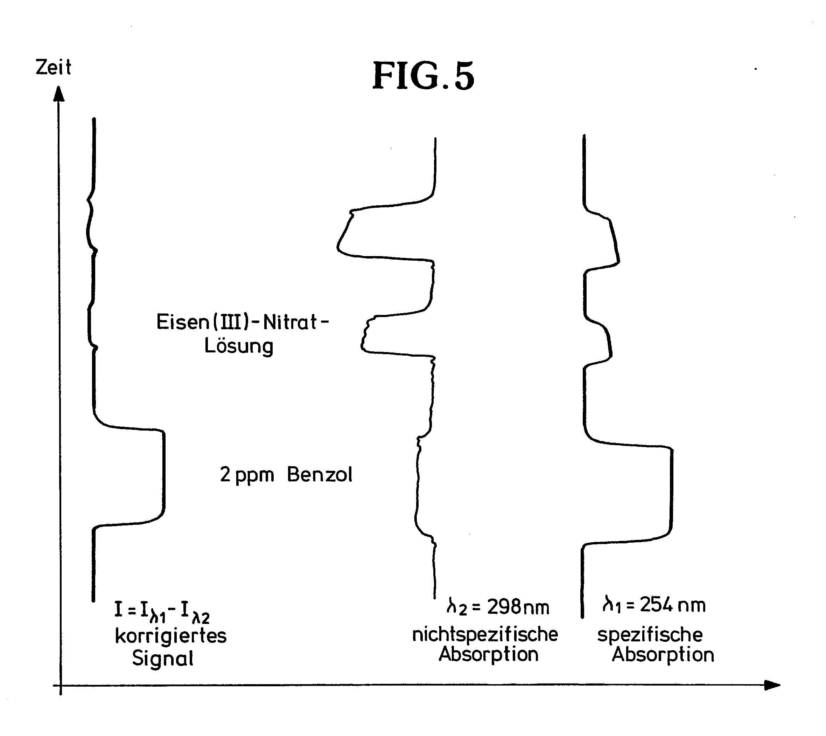

Figur 5 drei von einem Meßschreiber aufgezeichnete Meßkurven für Benzol in Wasser

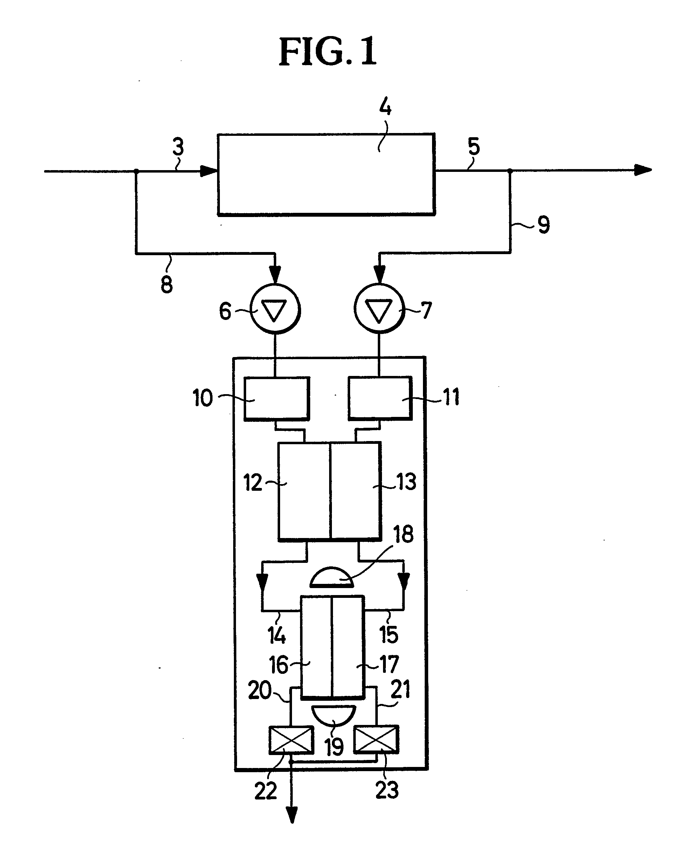

Die Vorrichtung gemäß Figur 1 besteht im wesentlichen aus einer vergleichsweise sauberes Eingangswasser führenden Rohrleitung 3, einem für einen Prozeß benötigten Rohrleitungsblock 4, einer das möglicherweise durch den Prozeß verunreinigte Ausgangswasser führenden Rohrleitung 5, den beiden Zweigleitungen 8, 9 und den in diese eingeschalteten Pumpen 6, 7, den beiden Blasenabscheidern 10, 11, den beiden den Blasenabscheidern 10, 11 nachgeschalteten Wärmetauschern 12, 13, den Zweigleitungen 14, 15, die an die Küvetten 16, 17 angeschlossen sind, der Lichtquelle 18 und dem Detektor 19, den beiden Abflußleitungen 20, 21 und schließlich den in die Abflußleitungen eingeschalteten Magnetventilen 22, 23.

Die Vorrichtung arbeitet wie folgt: Vom dem Rohrleitungsblock 4 zufließenden Eingangswasser wird aus der Rohrleitung 3 über die Zweigleitung 8 von der Pumpe 6 ein Wasserstrom abgezweigt, der dann in den Blasenabscheider 10 und von dort aus in den Wärmetauscher 12 gefördert wird. Der von den beiden Aggregaten 6 und 12 weitgehend blasenfrei gemachte, temperierte Wasserstrom wird nun durch die Küvette 16 und anschließend in die Abflußleitung 20 gefördert. Gleichzeitig wird vom Ausgangswasser über die Zweigleitung 9 ein zweiter Wasserstrom abgezweigt, der dann von der Pumpe 7 in den Blasenabscheider 11 und den Wärmetauscher 13 in die zweite Küvette 17 und anschließend in die Abflußleitung 21 gedrückt wird.

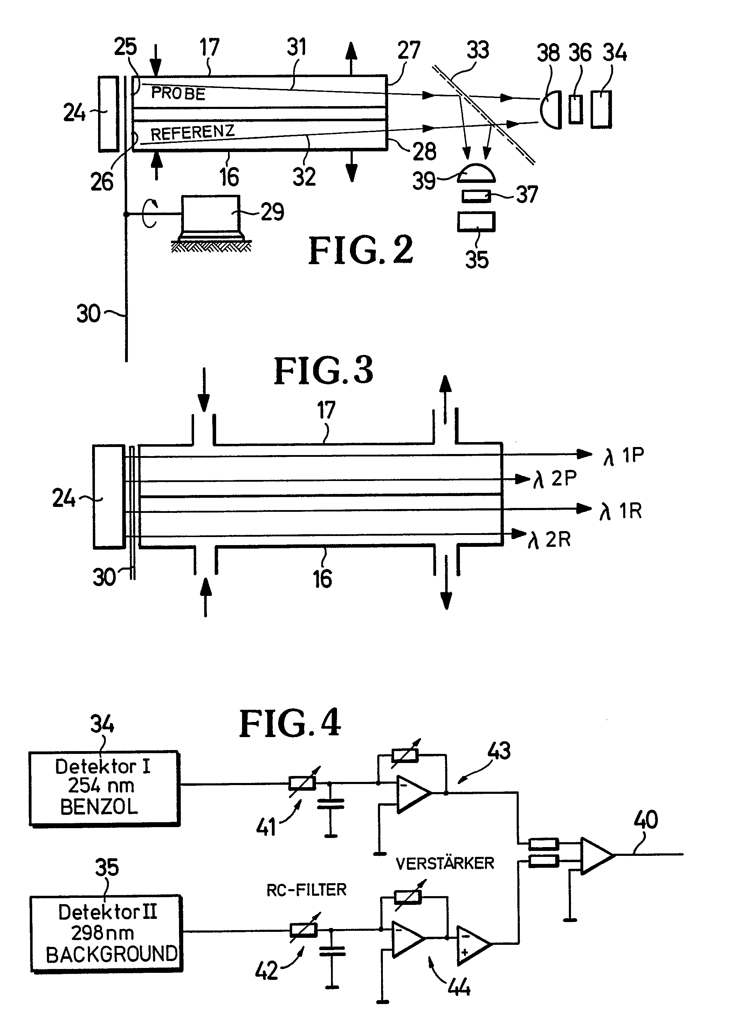

Wie aus Figur 2 ersichtlich, ist den beiden Küvetten 16, 17 eine UV-Lichtquelle 24 zugeordnet, deren Strahlen durch die Küvetten 16, 17 und das in diesen vorhandene Wasser hindurchdringen, da die vorderen und hinteren Stirnwände 25, 26 bzw. 27, 28 der Küvetten aus durchscheinendem Werkstoff gefertigt sind.

Zwischen den Küvetten 16, 17 und der UV-Lichtquelle 24 rotiert eine von einem Motor 29 angetriebene Lochscheibe 30 derart, daß die Strahlenbündel 31, 32 abwechselnd auf einen halbdurchlässigen Spiegel 33 fallen, der diese auf die Detektoren 34, 35 (die in Fig. 1 als ein Schaltsymbol 19 dargestellt sind) lenkt. Den Detektoren 34, 35 ist jeweils ein Lichtfilter 36 bzw. 37 für zum Beispiel 254 nm und 298 nm Wellenlänge vorgeschaltet. Weiterhin sind Sammellinsen 38 bzw. 39 vorgesehen, die die Strahlenbündel 31, 32 auf die Detektoren 34 bzw. 35 lenken. Die von den beiden Detektoren 34, 35 erzeugten elektrischen Signale werden in der aus Figur 4 ersichtlichen elektrischen Schaltung zu einem Signal aufbereitet, das an dem elektrischen Leiter 40 ansteht. Die Schaltung selbst weist RC-Filter 41, 42 und Verstärkerteile 43, 44 auf, die die von den Detektoren 34, 35 gelieferten elektrischen Signale aufbereiten.

Während die beiden Wasserströme von den Pumpen 6, 7 gleichmäßig durch die Küvetten 16, 17 gefördert werden, läuft das Chopper-Rad mit gleichbleibender Geschwindigkeit um, so daß pro Chopper-Umdrehung jeweils vier Meßwerte parallel ermittelt werden können:

spezifische Absorption in der Referenzseite: $\lambda 1 R$ ②
nichtspezifischer Absorption in der Referenzseite: $\lambda 2 R$ ④
spezifische Absorption in der Probenseite: $\lambda 1 P$ ①
nichtspezifische Absorption in der Probenseite: $\lambda 2 P$ ③

Der Chopper 30 gibt abwechslungsweise den Strahlengang zwischen Probenseite 17 und Referenzseite 16 frei, während die Signale für die spezifische Absorption $\lambda 1$ und die nichtspezifische Absorption $\lambda 2$ synchron durch zwei Detektoren 34, 35 ermittelt werden.

Dieses Beispiel zeigt, daß in der Referenz ④ eine unspezifische Absorption (nicht durch Benzol!) stattfindet.

Ohne spezifische Absorption müßte ③ und ④ gleich groß sein.

Daraus muß man schließen, daß ② ebenfalls mit dem Faktor ③ / ④ unterbewertet ist.

②' = ② • ③ / ④

Das eigentliche Signal ist also der Meßwert unter ①, von dem der korrigierte Referenzwert ②' abgezogen wurde.

Das korrigierte Signal errechnet sich also folgendermaßen:

$$\text{Signal} = \textcircled{1} - \textcircled{2} \cdot \frac{\textcircled{3}}{\textcircled{4}}$$

$$\hat{=} \frac{\dfrac{^{I}R_2}{^{I}R_1} - \dfrac{^{I}P_2}{^{I}P_1}}{\dfrac{^{I}R_2}{^{I}R_1}}$$

Auflistung der Einzelteile

3 Rohrleitung für Eingangswasser
4 Rohrleitungsblock
5 Rohrleitung für Ausgangswasser
6 Pumpe
7 Pumpe
8 Zweigleitung
9 Zweigleitung
10 Blasenabscheider
11 Blasenabscheider
12 Wärmetauscher
13 Wärmetauscher
14 Zweigleitung
15 Zweigleitung
16 Küvette
17 Küvette
18 Lichtquelle
19 Detektor
20 Abflußleitung
21 Abflußleitung
22 Magnetventil
23 Magnetventil
24 UV-Lichtquelle
25 vordere Stirnwand
26 vordere Stirnwand
27 hintere Stirnwand
28 hintere Stirnwand

29 Motor

30 Lochscheibe, Chopper

31 Strahlenbündel

32 Strahlenbündel

33 halbdurchlässiger Spiegel (Strahlenteiler)

34 Detektor

35 Detektor

36 Lichtfilter

37 Lichtfilter

38 Sammellinse

39 Sammellinse

40 elektrischer Leiter

41 RC-Filter

42 RC-Filter

43 Verstärker

44 Verstärker


**Ansprüche**

1. Vorrichtung zum Messen des Fremdstoffanteils in strömenden Flüssigkeiten, insbesondere zur Bestimmung der Konzentration von aromatischen Kohlenwasserstoffen in einem Wasserstrom, dadurch gekennzeichnet, daß jeweils vor und nach dem Leitungsblock (4) für den Prozeß ein Flüssigkeitsstrom über Leitungen (8, 9) abgezweigt und jeweils in eine Küvette (16, 17) gefördert wird, wobei den Küvetten (16, 17) jeweils eine Strahlenquelle (18) zugeordnet ist, deren Strahlenbündel (31, 32) die Küvetten (16, 17) durchdringen und von einem Strahlenteiler (33) jeweils sowohl einem ersten Detektor (34) als auch einem zweiten Detektor (35) zugeleitet werden, wobei jedem der Detektoren (34, 35) jeweils ein Strahlenfilter (36, 37) vorgeschaltet ist, das die Strahlen in zwei Strahlenbündel unterschiedlicher Wellenlängen aufteilt und wobei zwischen Strahlenquelle (24) und den beiden Küvetten ein rotierendes Chopper-Rad (30) angeordnet ist, das die Strahlen der Strahlenquelle (24) abwechselnd in die eine und die andere Küvette (16, 17) lenkt, wobei die elektrischen Signale der Detektoren (34, 35) von einer elektrischen Schaltung (40 bis 44) miteinander verglichen und zu einem Erkennungssignal verarbeitet werden.

2. Vorrichtung nach Anspruch 1, dadurch gekennzeichnet, daß das Filter einstückig mit dem Strahlenteiler ausgebildet ist.

3. Vorrichtung nach den Ansprüchen 1 oder 2, dadurch gekennzeichnet, daß die beiden Küvetten (16, 17) gleiche Abmessungen aufweisen und ihre Stirnwände (25, 26 bzw. 27, 28) aus durchscheinendem Werkstoff gebildet sind.

4. Vorrichtung nach den Ansprüchen 1, 2 oder 3, dadurch gekennzeichnet, daß den Küvetten (16, 17) jeweils Wärmetauscher (12, 13) vorgeschaltet sind, die die Temperaturen der beiden über die Leitungen (8, 9) geförderten Flüssigkeitsströme einander angleichen.

5. Vorrichtung nach einem oder mehreren der vorhergehenden Ansprüche, dadurch gekennzeichnet, daß hinter die Küvetten (16, 17) in die beiden Abflußleitungen (20, 21) Ventile (22, 23) eingeschaltet sind, die wechselweise und in rascher Folge geöffnet und geschlossen werden, so daß ein pulsierender Flüssigkeitsstrom in den Abflußleitungen (20, 21) erzeugt wird.

6. Vorrichtung nach einem oder mehreren der vorhergehenden Ansprüche, dadurch gekennzeichnet, daß der Strahlenteiler (33) die Strahlenbündel (31, 32) im Verhältnis 50 : 50 teilt und daß ein dem einen Detektor (34) vorgeschaltetes Filter (36) für den relevanten Wellenlängenbereich (von z.B. 254 nm) durchlässig ist und daß ein dem anderen Detektor (35) vorgeschaltetes Filter (37) für den relevanten Wellenlängenbereich (von z.B. 298 nm) durchlässig ist.

7. Vorrichtung nach einem oder mehreren der vorhergehenden Ansprüche, dadurch gekennzeichnet, daß die beiden Küvetten (16, 17) jeweils eine lichte Querschnittsfläche aufweisen, die größer bemessen ist als die Querschnittsfläche des jeweils zugeordneten Strahlenbündels (31, 32).

# FIG.1

FIG.2

FIG.3

FIG.4

FIG.5